Europäisches Patentamt

European Patent Office

Office européen des brevets

(11) Publication number: **0 044 615**
**B1**

## EUROPEAN PATENT SPECIFICATION

(45) Date of publication of patent specification: **30.05.84**

(21) Application number: **81302661.4**

(22) Date of filing: **15.06.81**

(51) Int. Cl.³: **C 12 Q 1/00,** C 12 Q 1/32,
C 12 Q 1/48

(54) Method for the analysis of triglycerides.

(30) Priority: **21.07.80 US 171112**

(43) Date of publication of application:
**27.01.82 Bulletin 82/04**

(45) Publication of the grant of the patent:
**30.05.84 Bulletin 84/22**

(84) Designated Contracting States:
**BE CH DE FR GB IT LI NL SE**

(56) References cited:
DE-B-2 834 705
FR-A-2 320 552
GB-A-2 026 692
US-A-3 703 591
US-A-4 001 089
US-A-4 019 961
US-A-4 066 508

CHEMICAL ABSTRACTS, vol. 88, nr. 21, 22nd
May 1978, page 266, abstract 148516k
Columbus, Ohio, US MOTT, GLEN E. et al.:
"Enzymic determination of triglycerides in
human and baboon serum triglycerides"

CHEMICAL ABSTRACTS, vol. 83, no. 1, 7th July
1975, page 387-388, abstract 4284g, Columbus,
Ohio, US G. BUCOLO et al.: "Lipase-triggered
kinetic assay of serum triglycerides"

(73) Proprietor: **TECHNICON INSTRUMENTS
CORPORATION**
**511 Benedict Avenue**
**Tarrytown, New York 10591 (US)**

(72) Inventor: **Leon, Luis**
**109 Buckboard Lane**
**Fairfield Connecticut 06430 (US)**
Inventor: **Ahmad, Syed I.**
**1 Wisteria Court**
**Orangeburg New York 10962 (US)**
Inventor: **Yeh, CHien-Kuo**
**38 Lake Shore Drive**
**Pleasantville New York 10570 (US)**

(74) Representative: **Wain, Christopher Paul et al**
**A.A. THORNTON & CO. Northumberland House**
**303-306 High Holborn**
**London WC1V 7LE (GB)**

(56) References cited:
CHEMICAL ABSTRACTS, vol. 93, no. 7, 18th
August 1980, page 443, abstract 65300n
Columbus, Ohio, US HINSCH W, et al.: "Fully
enzymic method of plasma triglyceride
determination using an immobilized glycerol
dehydrogenase nylon-tube reactor"

Courier Press, Leamington Spa, England.

**0 044 615**

## Description

This invention is concerned with the analytical enzymatic determination of serum triglycerides. We have devised an improved method of enzymatically determining serum triglycerides.

According to the invention, there is provided a method of analysis of triglycerides in a biological fluid which comprises the steps of (a) pretreating biological fluid to remove or compensate for interferences arising from endogenous glycerol and pyruvate, (b) enzymatically hydrolyzing the triglycerides in the resulting fluid by combining said fluid with a mixture comprising a microbial lipase, a surfactant which promotes said hydrolysis, and an alkaline earth metal cation, in the form of a salt, and (c) determining the amount of glycerol produced by said enzymatic hydrolysis, characterised in that the surfactant is selected from polyoxyethylene (POE) alkylamides, POE esters of fatty acids, POE mercaptans, POE alkylamines, POE polyol esters, POE acetylenic glycols and POE phosphate esters.

In the determination of triglycerides in biological fluids such as serum, substances such as glycerol and pyruvate which are endogenous to serum tend to interfere in the assay, and to overcome this interference it is necessary to remove these substances or to compensate for them, e.g. by carrying out the so-called parallel blank correction. This correction procedure can be time-consuming.

According to a highly preferred feature of the present invention, we have found that interference from endogenous glycerol and pyruvate can be avoided, and the necessity for parallel blank corrections eliminated by removing these substances enzymatically in step (a). In one preferred such enzymatic removal, step (a) of the method of the invention is as follows:

(a) treating said biological fluid to remove endogenous glycerol and pyruvate by combining with said fluid, as a first component: adenosine triphosphate (ATP), the enzyme glycerol kinase (GK) and a metal cation activator for said GK; as a second component: enzyme pyruvate kinase (PK) in the presence of substrate phosphoenol pyruvate (PEP), and an enzyme PK activator; and as a third component: glutamate and enzyme alanine amino-transferase (GPT)

Alternatively, step (a) can employ a substrate-enzyme system selected from: acetyl kinase and acetyl phosphate, creatine kinase and creatine phosphate, 3-phosphoglycerate kinase and glycerate-1,3-diphosphate.

In another possibility, step (a) uses the enzyme glycerol dehydrogenase or glycerol oxidase.

The method of the invention is particularly, but not exclusively, useful in the analysis of body fluids such as serum.

In the preferred method of the invention, step (a), the first component enzyme is glycerol kinase (GK) and the second component enzyme is pyruvate kinase (PK). The following more detailed description of the invention refers mainly to the use of these preferred enzymes but it is to be understood that the description applies also to the use of other enzymes, *mutatis mutandis.*

In accordance with a preferred aspect of the invention, the removal of glycerol and pyruvate interferences endogenous to the biological fluid is accomplished pursuant to the following reaction scheme:

$$\text{glycerol (endogenous)} + \text{ATP} \xrightarrow[\text{Mn}^{++}]{\text{GK}} \text{glycerol phosphate} + \text{ADP}$$

$$\xrightarrow[\text{K}^{+}]{\text{PK}}$$

PEP

pyruvate

+

$$\text{endogenous pyruvate} \xrightarrow{\text{GPT}} \text{alanine}$$

+ +

glutamate $\alpha$-ketoglutarate

2

0 044 615

In the above, ATP is adenosine triphosphate, ADP is adenosine diphosphate, and PEP is phosphoenol pyruvate. As can be seen, the endogenous glycerol is completely converted to the phosphorylated form by the action of a specific kinase, e.g. glycerol kinase (GK). (This is kinase reaction (1).) The phosphorylated glycerol does not interfere in the analysis. The ADP produced is recycled back to ATP under the influence of a specific kinase, e.g. pyruvate kinase (PK), and in the presence of PEP. (This is kinase reaction (2).). This latter reaction is needed to eliminate the ADP which will interfere in the accurate quantitation of triglycerides (TG). In the above reaction-scheme, the endogenous pyruvate together with the pyruvate produced in the previous reaction, in the presence of glutamate, is transformed to alanine and $\alpha$-ketoglutarate which do not interfere in the TG quantitation.

As will be understood, it is necessary to stop the kinase reaction (1) after all the endogenous glycerol has been transformed to glycerol-phosphate. Failure to stop reaction (1) will result in the conversion of glycerol (produced by lipolysis) to glycerol-1-phosphate with concomitant production of ADP. There are two general ways of stopping of kinase reaction (1):

(i) A metal chelating compound is used to sequestrate the specific metal ion used to activate the specific kinase (1). Once the metal ion is chelated (e.g. by ethylenediamine tetraacetic acid (EDTA)), the kinase (1) is completely rendered inactive.

(ii) The kinase (1) enzyme can be used in immobilized form, e.g. physically attached to the inner wall of a plastics (e.g. nylon) conduit, and the lipolysis reaction effected out of contact with the kinase enzyme (1).

For the above illustrated reaction scheme, the preferred formulations are as follows:

*Reagent No. 1*

This reagent contains glycerol kinase, pyruvate kinase, alanine aminotransferase and ATP. The reagent will also contain a buffer system and the required enzyme activators. Other reagent formulations have been investigated and found to give equivalent results. Table No. 1 illustrates the enzymes and substrates which could be used as replacements for the specific kinase (2) reaction.

TABLE NO. 1

| Enzyme | Substrate |
| --- | --- |
| Acetyl Kinase | Acetyl Phosphate |
| Creatine Kinase | Creatine Phosphate |
| 3-Phosphoglycerate Kinase | Glycerate-1,3-Diphosphate |

*Reagent No. 2*

This reagent is used to stop the activity of the kinase (1) as discussed in approach (i) above.

The reagent responsible for the enzymatic hydrolysis of triglycerides comprises a mixture of microbial lipase (e.g. Candida Cylindracea), a surfactant (e.g. a polyoxyethylene alkanolamide) and a calcium salt, e.g. $CaCl_2$. All three components are essential for complete hydrolysis. The microbial lipase may be obtained from a culture broth of *Candida Cylindracea*. Such Candida lipase should have an activity within the range of 40,000 u/l. to 1,000,000 u/l. (reaction mix). Other Candida lipases were found to give equivalent results when used in combination with the surfactant and $CaCl_2$.

As indicated above, a surfactant which promotes the hydrolysis is an essential component of the triglyceride-hydrolysis mixture. While we prefer to use a surfactant belonging to the non-ionic group of polyoxyethylene alkanolamides, the following other non-ionic surfactant groups also contain members which give equivalent results:

1. Polyoxyethylene (POE) ester of fatty acids

2. POE mercaptans

3. POE alkylamides

4. POE alkylamines

5. POE polyol esters

6. POE acetylenic glycols

7. POE phosphate esters.

The general reaction sequences for the quantitation of glycerol produced by lipolysis are summarised by the following reaction scheme.

3

$$\text{glycerol} \quad + \quad \text{ATP} \quad \xrightarrow[\text{Mg}^{++}]{\text{glycerol kinase}} \quad \text{glycerol-1-phosphate} \quad + \quad \text{ADP}$$

$$\text{ADP} \quad + \quad \text{PEP} \quad \xrightarrow[\text{K}^{+}]{\text{pyruvate kinase}} \quad \text{ATP} \quad + \quad \text{pyruvate}$$

$$\text{pyruvate} \quad \xrightarrow{\text{LDH}} \quad \text{lactate}$$

$$\text{NADH} \qquad \qquad \text{NAD}^{+}$$

In the above scheme, the glycerol produced by lipolysis is dialyzed into a buffered reagent containing PEP, ATP Mg$^{++}$ and NADH. The glycerol kinase, pyruvate kinase and lactate dehydrogenase (LDH) reactions are advantageously effected inside an immobilized enzyme coil (see flow diagram above) where the three latter enzymes are bound to the inner wall of nylon tubing. Also, inside the immobilised enzyme coil, NADH is oxidized to NAD. The decrease in absorbance of NADH to NAD is directly proportional to the amount of glycerol produced in the lipolysis step.

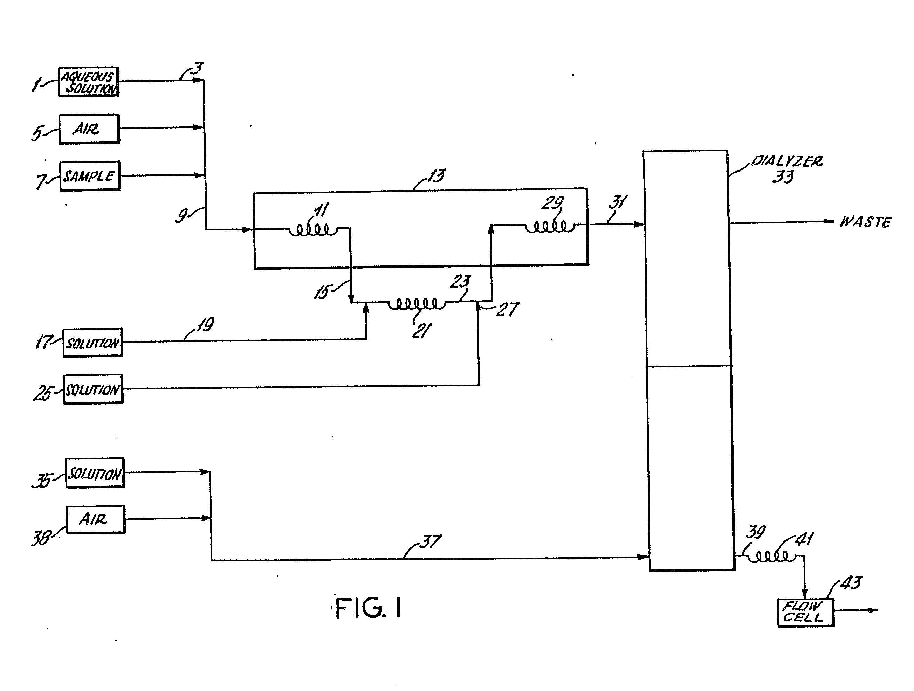

The accompanying Figure represents a schematic flow sheet of a continuous system or apparatus according to the present invention for the analysis of triglycerides in a biological fluid.

Referring to the Figure, an aqueous solution 1 which contains GK, PK, GPT, ATP, glutamate, PEP, Mn$^{++}$ and K$^{+}$ is pumped into conduit 3 where air 5 is added followed by sample 7. The mixed solution is conveyed via line 9 and passed through coil 11 in heating bath 13 which is maintained at a temperature of about between 30°C and 50°C. Coil 11 may be fabricated of a plastics material such as nylon, polyethylene, polyvinyl chloride or polystyrene, or for example of another material such as aluminium, glass, stainless steel or silicon rubber. Nylon is preferred. As the solution passes through coil 11, removal of endogenous glycerol and pyruvate occurs. The treated solution is conveyed via line 15 where solution 17 containing EDTA and surfactant is pumped via line 19 and combines with the solution which then flows through coil 21 which is at ambient temperature. On passing through coil 21, enzyme GK is inactivated. After the solution passes through coil 21, it is conveyed via line 23 and is combined with a solution 25 containing microbial lipases and a surfactant at 27. The combined solution is then passed through coil 29 in heating bath 13 where enzymatic hydrolysis occurs. The resulting solution is conveyed via line 31 to dialyzer 33 where the dialyzed solution is conveyed to the bottom portion of the dialyzer and combined with solution 35 which contains NADH and Mg$^{++}$ in solution via line 37 to which air 38 is also conveyed. The resulting solution is conveyed via line 39 to coil 41 where enzymes glycerol kinase (GK), pyruvate kinase (PK) and lactate dehydrogenase (LDH) are immobilised. After passing through coil 41, the resulting solution is photometrically scanned at 340 nm in flowcell 43.

In another embodiment of this invention, the above system is modified so that coil 11 contains immobilised enzyme GK. In this embodiment, no EDTA is necessary. As to other embodiments, coil 11 may also contain immobilised enzyme PK and/or immobilised enzyme GPT.

Example 1

The following procedure illustrates one manner of carrying out the present invention in a continuous manner as in the Technicon AutoAnalyzer biochemical analyzer as described in U.S. patent No. 2,797,149. ("Technicon" and "AutoAnalyzer" are trade marks).

The human serum sample (0.020—0.028 ml.) is combined with reagent No. 1 which contains glycerol kinase (50—200 units/dl.), ATP (5—16.5 mM.), MnCl$_2$ (1—20 mM.), pyruvate kinase (50—1000 units/dl.), PEP (5—16 mM.), CaCl$_2$ (30—60 mM.), GPT (50—500 units/dl.), glutamate (50—30 mM.) and tris buffer (50—100 mM, pH 7.6).

After mixing for 1/2 to 2 minutes at approximately 30 to 50°C, the resultant solution is mixed with reagent No. 2 which contains EDTA (40—80 mM.), 5-POE-lauricamide (7—15 g./l.) and tris buffer (50—100 mM., pH 7.5). After mixing for 10—60 seconds at room temperature, the resulting solution is

**0 044 615**

is mixed with reagent No. 3 which contains lipase (100,000—2,000,000 units/1.), bovine serum albumin (.1—2 g/dl.), CaCl$_2$ (40—200 mM), and maleate buffer (50—100 mM, pH 6).

After mixing for 1 to 10 minutes at approximately 30—50°C, the resulting solution is dialyzed across a dialyzer block equipped with a semi-permeable membrane.

The dialyzed glycerol is combined with an aqueous buffer solution of pH 7.8 which contains reduced nicotinamide-adeninedinucleotide (NADH) (70—140 mg/l.), MgSO$_4$ (2—6 mM) and KCl (20—60 mM) and this solution is passed through an immobilised enzyme coil to which is physically bound GK, PK and LDH. After passing through the coil, the decease of the NADH absorbance is monitored at 340 nm.

Example II

The procedure of Example I is repeated except that only enzyme GK is immobilised. In this procedure no EDTA is used.

Example III

The procedure of Example I is repeated except that MgCl$_2$ is used in place of MnCl$_2$.

Example IV

The procedure of Example II is repeated wherein enzyme PK is employed along with GK in immobilised nylon coil form.

Example V

The procedure of Example II is repeated except that enzyme GPT is employed along with enzyme GK in immobilised nylon coil form.

Example VI

The procedure of Example I is repeated except that the following surfactants are used in place of POE-5 lauric acid:

POE-5 cocoamide

15-POE cocoamine.

Example VII

The procedure of Example I is repeated except that the following enzymes are used in place of GK:

glycerol dehydrogenase

glycerol oxidase.

In this procedure, the above enzymes together with enzyme GPT are in immobilised form in the same coil. No enzyme PK is necessary.

Example VIII

The procedure of Example II is repeated wherein in place of the PEP-PK system, the following substrate-enzyme systems are used:

acetyl phosphate-acetyl kinase

creatine phosphate-creatine kinase

glycerate 1,3-diphosphate-3-phosphoglycerate kinase.

## Claims

1. A method of analysis of triglycerides in a biological fluid which comprises the steps of (a) pretreating biological fluid to remove or compensate for interferences arising from endogenous glycerol and pyruvate, (b) enzymatically hydrolyzing the triglycerides in the resulting fluid by combining said fluid with a mixture comprising a microbial lipase, a surfactant which promotes said hydrolysis, and an alkaline earth metal cation, in the form of a salt, and (c) determining the amount of glycerol produced by said enzymatic hydrolysis, characterised in that the surfactant is selected from polyoxyethylene (POE) alkylamides, POE esters of fatty acids, POE mercaptans, POE alkylamines, POE polyol esters, POE acetylenic glycols and POE phosphate esters.

2. A method according to claim 1 wherein, in step (a), the said interferences are removed enzymatically by a process characterized by combining with said biological fluid, as a first component:

5

adenosine triphosphate (ATP), the enzyme glycerol kinase (GK) and a metal cation activator for said GK; as a second component: enzyme pyruvate kinase (PK) in the presence of substrate phospoenol pyruvate (PEP), and an enzyme PK activator; and as a third component: glutamate and enzyme alanine amino-transferase (GPT); and rendering said cation ineffective subsequent to removal of said endogenous glycerol.

3. A method according to claim 1 wherein, in step (a), the said interferences are removed enzymatically by a process characterised by combining with said biological fluid, as a first component: adenosine triphosphate (ATP), the enzyme glycerol kinase (GK) and a metal cation activator for said GK; as a second component: enzyme pyruvate kinase (PK) in the presence of substrate phosphoenol pyruvate (PEP), and an enzyme PK activator; and as a third component: glutamate and enzyme alanine amino-transferase (GPT); wherein said enzyme GK is employed in immobilised form in which said enzyme is physically attached to a portion of the inner wall of a conduit through which the fluid passes in contact with the enzyme, and said other enzymes PK and GPT are optionally physically attached to the same conduit.

4. A method according to claim 3, characterised in that the coil is composed of nylon, polyethylene polyvinyl chloride or polystyrene; or aluminium, glass, stainless steel or silicon rubber.

5. A method according to any preceding claim, characterised in that wherein said microbial lipase is a a Candida lipase.

6. A method according to claim 5, characterised in that said Candida lipase is *Candida Cylindracea* having an activity in the range of 40,000 u/l. to 1,000,000 u/l.

7. A method according to any preceding claim, characterised in that said surfactant is POE-5-lauricamide or POE-5-cocamide.

8. A method according to any preceding claim, characterised in that said glycerol determination step (c) comprises dialyzing the reaction product from said enzymatic hydrolysis into a buffered reagent comprising PEP, ATP, magnesium ion in the form of a salt, and NADH, and passing said dialyzed solution through an immobilised enzyme coil to which is physically bound enzymes glycerol kinase (GK), pyruvate kinase (PK) and lactate dehydrogenase (LDH) and photometrically measuring at 340 nm the decrease in absorbance of NADH to NAD.

9. A method according to claim 1, characterised in that in step (a) the pretreatment employs a substrate-enzyme system selected from acetyl phosphate-acetyl kinase, creatine phosphate-creatine kinase and glycerate 1,3-diphosphate-3-phosphoglycerate kinase.

10. A method according to claim 1, characterised in that in step (a) the pretreatment employs an enzyme selected from glycerol dehydrogenase and glycerol oxidase.

## Patentansprüche

1. Verfahren zur Analyse von Triglyceriden in einem biologischen Fluid, wobei man (a) das biologische Fluid einer Vorbehandlung zur Beseitigung oder zum Ausgleich von Störungen unterwirft, die von endogenem Glycerin und Pyruvat hervorgerufen werden, (b) die Triglyceride in dem erhaltenen Fluid enzymatisch hydrolysiert, indem man das Fluid mit einem Gemisch vereinigt, das eine mikrobielle Lipase, ein oberflächenaktives Mittel als Promotor für die Hydrolyse und ein Erdalkalikation in Form eines Salzes enthält, und (c) die Menge an Glycerin, die durch diese enzymatische Hydrolyse erzeugt worden ist, bestimmt, dadurch gekennzeichnet, daß man als oberflächenaktives Mittel Polyoxyethylenalkylamide, Polyoxyethylenester von Fettsäuren, Polyoxyethylenmercaptane, Polyoxyethylen-alkylamine, Polyoxyethylenpolyolester, acetylenische Polyoxyethylenglycole, Polyoxyethylen-phosphatester und Polyoxyethylenalcanolamide auswählt.

2. Verfahren gemäß Anspruch 1, dadurch gekennzeichnet, daß man in Stufe (a) die Störungen enzymatisch durch ein Verfahren beseitigt, bei dem man mit dem biologischen Fluid als erste Komponente Adenosintriphosphat (ATP), das Enzym Glycerinkinase (GK) und ein Metallkation als Aktivator für die Glycerinkinase; als zweite Komponente das Enzym Pyruvatkinase (PK) in Gegenwart des Substrates Phosphoenolpyruvat (PEP) und einen Aktivator für das Enzym Pyruvatkinase; und als dritte Komponente Glutamat und das Enzym Alaninaminotransferase (GPT) vereinigt und nach der Entfernung des endogenen Glycerins das Kation unwirksam macht.

3. Verfahren gemäß Anspruch 1, dadurch gekennzeichnet, daß man in Stufe (a) die Störungen enzymatisch nach einem Verfahren beseitigt, bei dem man mit dem biologischen Fluid als erste Komponente Adenosintriphosphat (ATP), das Enzym Glycerinkinase (GK) und ein Metallkation als Aktivator für die Glycerinkinase; als zweite Komponente das Enzym Pyruvatkinase (PK) in Gegenwart des Substrates Phosphoenolpyruvat (PEP) und einen Aktivator für das Enzym Pyruvatkinase; und als dritte Komponente Glutamat und das Enzym Alaninaminotransferase (GPT) vereinigt, wobei das Enzym Glycerinkinase in unbeweglich gemachter Form verwendet wird, in der es physikalisch an einen Abschnitt der Innenwand der Leitung angeheftet ist, durch die das Fluid in Kontakt mit dem Enzym hindruchströmt, und die anderen Enzyme, Pyruvatkinase und Alaninaminotransferase, gewünschtenfalls ebenfalls physikalisch an dieselbe Leitung angeheftet sind.

4. Verfahren gemäß Anspruch 3, dadurch gekennzeichnet, daß man eine Leitung oder Mischschlange verwendet, die aus Polyamid (Nylon), Polyethylen, Polyvinylchlorid oder Polystyrol oder aus Aluminium, Glas, rostfreiem Stahl oder Siliconkautschuk besteht.

5. Verfahren gemäß einem der vorhergehenden Ansprüche, dadurch gekennzeichnet, daß man als mikrobielle Lipase eine Candidalipase verwendet.

6. Verfahren gemäß Anspruch 5, dadurch gekennzeichnet, daß man als Candidalipase Candida-Cylindracea mit einer Aktivität im Bereich von 40000 bis 1000000 Einheiten pro Liter verwendet.

7. Verfahren gemäß einem der vorhergehenden Ansprüche, dadurch gekennzeichnet, daß man als oberflächenaktives Mittel Polyoxyethylen-5-Laurinsäureamid oder Polyoxyethylen-5-Kokosfettsäuren-amid verwendet.

8. Verfahren gemäß einem der vorhergehenden Ansprüche, dadurch gekennzeichnet, daß man in der Glycerinbestimmungsstufe (c) das Reaktionsprodukt der enzymatischen Hydrolyse in ein gepuffertes Reagenz dialysiert, das Phosphoenolpyruvat (PEP), Adenosintriphosphat (ATP), Magnesiumionen in Form eines Salzes und NADH enthält, daß man die dialysierte Lösung durch eine Mischschlange mit immobilisiertem Enzym leitet, an die die Enzyme Glycerinkinase (GK), Pyruvatkinase (PK) und Lactatdehydrogenase (LDH) physikalisch angeheftet sind, und daß man bei 340 nm photometrisch die Umwandlung von NADH in NAD durch den Abfall der Absorption mißt.

9. Verfahren gemäß Anspruch 1, dadurch gekennzeichnet, daß man in Stufe (a) zur Vorbehandlung ein Substrat/Enzym-System verwendet, das aus Acetylphosphat/Acetylkinase, Greatinphosphat/Creatinkinase und Glycerat-1, 3-Diphosphat/3-Phosphoglyceratkinase verwendet.

10. Verfahren gemäß Anspruch 1, dadurch gekennzeichnet, daß man in Stufe (a) zur Vorbehandlung als Enzym Glycerindehydrogenase und bzw, oder Glycerinoxydase verwendet.

**Revendications**

1. Un procédé d'analyse des triglycérides dans un liquide biologique qui comprend les stades de (a) prétraitement du liquide biologique pour éliminer ou compenser les interférences dues au glycérol et au pyruvate endogènes, (b) hydrolyse enzymatique des triglycérides dans le liquide obtenu par combinaison dudit liquide avec un mélange comprenant une lipase microbienne, un agent tensio-actif qui favorise ladite hydrolyse et un cation de métal alcalino-terreux, sous forme d'un sel, et (c) détermination de la quantité de glycérol produite par ladite hydrolyse enzymatique, caractérise en ce que l'agent tensio-actif est choisi parmi les polyoxyéthylène (POE) alkylamides, les POE-esters d'acides gras, les POE-mercaptans, les POE-alkylamines, les POE-esters de polyols, les POE-glycols acétyléniques et les POE-esters phosphoriques.

2. Un procédé selon la revendication 1 dans lequel, dans le stade (a), lesdites interférences sont éliminées par voie enzymatique selon un procédé caractérisé par la combinaison avec ledit liquide biologique, comme premier composant: d'adénosinetriphosphate (ATP), de l'enzyme glycérol-kinase (GK) et d'un cation métallique activateur de ladite GK; comme second composant: de l'enzyme pyruvate-kinase (PK) en présence du substrat phospho-énolpyruvate (PEP) et d'un activateur de l'enzyme PK; et comme troisième composant: du glutamate et de l'enzyme alanine-amino-transférase (GPT); et l'inactivation dudit cation après l'élimination dudit glycérol endogène.

3. Un procédé selon la revendication 1 dans lequel, dans le stade (a) lesdites interférences sont éliminées par voie enzymatique selon un procédé caractérisé par la combinaison avec ledit liquide biologique, comme premier composant: d'adénosinetriphosphate (ATP), de l'enzyme glycérol-kinase (GK) et d'un cation métallique activateur de ladite GK; comme second composant: de l'enzyme pyruvate-kinase (PK) en présence du substrat phospho-énolpyruvate (PEP) et d'un activateur de l'enzyme PK; et comme troisième composant: du glutamate et de l'enzyme alanine-amino-transférase (GPT); où ladite enzyme GK est utilisée sous une forme immobilisée dans laquelle ladite enzyme est fixée physiquement à une portion de la paroi intérieure d'une canalisation à travers laquelle le liquide passe en contact avec l'enzyme et lesdites autres enzymes PK et GPT sont éventuellement attachées physiquement à la même canalisation.

4. Un procédé selon la revendication 3, caractérisé en ce que la bobine est composée de nylon, de polyéthylène, de chlorure de polyvinyle ou de polystyrène; ou d'aluminium, de verre, d'acier inoxydable ou de caoutchouc de silicone.

5. Un procédé selon l'une quelconque des revendications précédentes, caractérisé en ce que ladite lipase microbienne est une lipase de Candida.

6. Un procédé selon la revendication 5, caractérisé en ce que ladite lipase de Candida est Candida Cylindracea ayant une activité dans la gamme de 4 000 u/l à 1 000 000 u/l.

7. Un procédé selon l'une quelconque des revendications précédentes, caractérisé en ce que ledit agent tensio-actif est le POE-5-lauricamide ou le POE-5-cocoamide.

8. Un procédé selon l'une quelconque des revendications précédentes, caractérisé en ce que ledit stade de détermination du glycérol (c) comprend la dialyse du produit réactionnel de ladite hydrolyse enzymatique dans un réactif tamponné comprenant du PEP, de l'ATP, l'ion magnésium sous la forme d'un sel et du NADH et le passage de ladite solution dyalisée à travers une bobine d'enzyme immobilisée à laquelle sont liées physiquement les enzymes glycérol-kinase (GK), pyruvate-kinase (PK) et lactate-déshydrogénase (LDH) et la mesure photométrique à 340 nm de la diminution de l'absorbance du NADH en NAD.

9. Un procédé selon la revendication 1, caractérisé en ce que dans le stade (a) le prétraitement utilise un système substrat-enzyme choisi parmi acétylphosphate-acétylkinase, créatinephosphate-créatinekinase et glycérate-1,3-diphosphate-3-phosphoglycérate-kinase.

10. Un procédé selon la revendication 1, caractérisé en ce que dans le stade (a) le prétraitement utilise une enzyme choisie parmi la glycérol-déshydrogénase et la glycérol-oxydase.

AQUEOUS SOLUTION 1

AIR 5

SAMPLE 7

9

11

13

15

29

31

27

23

21

19

SOLUTION 17

SOLUTION 25

SOLUTION 35

AIR 38

37

39

41

43

FLOW CELL

DIALYZER 33

WASTE

FIG. I